# NEUE EUROPÄISCHE PATENTSCHRIFT

(11) **EP 0 253 198 B2**
(45) Veröffentlichungstag und Bekanntmachung des Hinweises auf die Entscheidung über den Einspruch: **23.06.1999**
(45) Hinweis auf die Patenterteilung: 26.09.1990
(21) Anmeldenummer: 87109374.6
(22) Anmeldetag: 30.06.1987
(51) Int. Cl.: A61L 25/00

(54) **Einkomponenten-Gewebekleber sowie Verfahren zu seiner Herstellung**
Single-component tissue adhesive and method for producing same
Colle pour tissu à un composant et son procédé de fabrication

(30) Priorität: 05.07.1986 DE 3622642
(43) Veröffentlichungstag der Anmeldung: 20.01.1988
(73) Patentinhaber: Centeon Pharma GmbH, 35002 Marburg (DE)
(72) Erfinder: Heimburger, Norbert, Prof. Dr., D-3550 Marburg (DE); Fuhge, Peter, Dr., D-3551 Lahntal (DE); Ronneberger, Hansjörg, Dr., D-3550 Marburg (DE)

(56) Entgegenhaltungen:
- EP-A- 0 068 047
- EP-A- 0 183 674
- FR-A- 2 448 901
- GB-A- 2 041 942
- Surgery, Band 97, Nr. 6, 1985, S. 750-755
- Transfusion, Band IX, Nr. 4, 1966, S. 303-319

## Beschreibung

Die Erfindung betrifft einen Gewebekleber, der Fibrinogen, Faktor XIII, einen Thrombininhibitor, Prothrombinfaktoren, Calciumionen und gegebenenfalls einen Plasmininhibitor enthält. Durch an der zu verklebenden Wunde natürlicherweise vorhandene Acceleratoren wird aus dem Prothrombin des Klebers das zur Klebung nötige Thrombin freigesetzt.

Als Ergebnis der physiologischen Vorgänge bei der Reparation von Läsionen menschlicher oder tierischer Gewebe schlägt sich im Verletzungsbereich Fibrin nieder. Dies wird dadurch ausgelöst, daß aus den verletzten Zellen Thromboplastin ausströmt, das beim Kontakt mit dem Plasmafaktor VII den Faktor-X-Aktivator bildet, der dann zusammen mit dem Faktor V und im Komplex mit Phospholipiden und Calcium Prothrombin in Thrombin umwandelt. Unter der Wirkung von Thrombin wiederum entstehen über die Abspaltung der Fibrinopeptide A und B aus dem Fibrinogen Fibrinmonomere, die durch Aggregation Fibrinstränge bilden, die vom F XIIIa, einer Transglutaminase, die ebenfalls durch Thrombin aktiviert wird, unter Bildung von Isopeptidbindungen kovalent vernetzt werden. Ein Inhibitor, nämlich das al` pha₂-Antiplasmin, wird vom Faktor XIII an die Fibrinstränge gebunden und schützt diese vor dem Abbau durch Plasmin.

Dieser Ablauf der Hämostase und Wundheilung läßt erkennen, daß neben dem Fibrinogen als Substrat zwei Enzyme erforderlich sind: das Thrombin und der Faktor XIII.

Dabei ist es Aufgabe des Thrombins, sowohl das Fibrinogen als auch den Faktor XIII zu aktivieren, das heißt in ihre reaktionsfähige Form umzuwandeln. Das ist im Falle des Fibrinogens das Fibrinmonomer und im Falle des Faktors XIII die katalytisch aktive Transglutaminase (F XIIIa).

Aus EP 0 068 047 ist eine angereicherte Plasmafraktion bekannt, die als Wundverschluß geeignet ist und Fibrinogen, einen Fibrinolyse-Inhibitor und Thrombin oder Prothrombin in einem wasserfreien System enthält. Dieser Kleber kann nicht in Wasser aufgelöst sondern nur als Pulver appliziert werden, was für die Anwendung von Nachteil ist. Wenn das Gemisch in Wasser gelöst wird, reagieren die Komponenten miteinander und es entsteht ein Gerinnsel. Aus den allgemeinen Angaben waren keine Anregungen zur Lösung der hier gestellten Aufgabe zu entnehmen.

Es ist für einen Einkomponenten-Kleber mit Wasser als Lösemittel schwierig, die Wirksubstanzen so und in solchen Mengenverhältnissen zu kombinieren, daß während der Herstellung des Klebers, der Rekonstitution mit einem Lösungsmittel und der Bereithaltung für die Anwendung keine vorzeitige und unerwünschte Aktivierung erfolgt und zum anderen aber nach dem Aufbringen auf die Klebestelle eine genügend hohe Festigkeit relativ schnell erreicht wird.

Überrraschenderweise wurden Bedingungen gefunden, die eine reproduzierbare Herstellung eines praktische handhabbaren Einkomponentenklebers in einem physiologischen Millieu ermöglichen.

Vorraussetzung für die Herstellung und Verarbeitungsfähigkeit eines solchen Einkomponentenklebers sind optimal aufeinander abgestimmte Konzentrationen von Fibrinogen, Plasmininhibitor, F XIII, Prothrombinkonzentrat, Thrombininhibitor und Calciumionen. Das trifft im besonderen Maße für AT III als Thrombininhibitor und Calciumionen zu, da die Calciumionen für die Aktivierung der Gerinnungsfaktoren essentiell sind. Hohe Konzentrationen beschleunigen die Thrombinbildung, kleine verzögern sie. Daher ist eine optimale Konzentration von Calciumionen erforderlich. Da sich bei einer solchen die Bildung von Thrombin nicht ausschließen läßt, ist die Kombination mit einem Thrombininhibitor, vorzugsweise AT III zweckmäßig. Denn einerseits muß bei der Herstellung des Klebers die Bildung von Thrombin ausgeschlossen werden, andererseits muß der Kleber schnell ein festes Gerinnsel bilden, wenn beim Kontakt mit Wundoberflächen die Gerinnung, das heißt die Abscheidung eines Fibrinfilms ausgelöst wird. Demzufolge ist die Herstellung eines Thrombin-haltigen Einkomponentenklebers und seine Anwendung in wässriger Lösung nicht denkbar, und auch Prothrombin läßt sich nur unter Bedingungen verwenden, die eine Aktiverung zu Thrombin während der Herstellung und Vorbereitung zur Anwendung verhindern. Dazu gehört die Einstellung bestimmter Konzentrationen an Thrombininhibitor und Calciumionen.

Gegenstand der Erfindung ist deshalb ein Gewebekleber, enthaltend in wässriger Lösung Fibrinogen, F XIII, einen Thrombininhibitor, Prothrombinfaktoren, Calciumionen und gegebenenfalls einen Plasmininhibitor.

Es war überraschend, daß Fibrinogen zusammen mit Prothrombin als sogenannte hypercoagulabile Lösung, die zudem noch Calciumionen und AT III enthält, abgefüllt und als Gewebekleber verwendet werden kann.

Ein solcher erfindungsgemäßer Kleber kann in eine feste Form gebracht, beispielsweise lyophilisiert, und mit Wasser rekonstituiert werden. Er kann alle Wirksubstanzen in pasteurisierter Form enthalten und ist dann frei vom Risiko einer Hepatitis- und HTLV III-Übertragung.

Das Mischungsverhältnis der Wirksubstanzen Fibrinogen, Plasmininhibitor, F XIII, Prothrombinfaktoren, Calciumionen und AT III ist derart, daß keine vorzeitige Aktivierung auftritt, daß aber das Gemisch beim Kontakt mit Wundoberflächen spontan gerinnt und Fibrin abscheidet.

Die Zeit in der dies geschieht, entspricht den klinischen Erfordernissen. Der erfindungsgemäße Einkomponentenkleber unterscheidet sich in seiner Wirkung nicht von den Zweikomponenten-Systemen des Standes der Technik.

Die Wirksubstanzen sind in diesem Kleber so gemischt daß in der Anwendungslösung die Konzentration von Fibrinogen bei 70 - 90 mg/ml und die der Prothrombinfaktoren zwischen 10 und 30 E/ml, bezogen auf F II, liegt. Die Konzentration an Calciumionen, die im Kleber zum Teil gebunden an Protein vorliegen, soll in der Anwendungslösung bei 0,5 - 1 mmol/l liegen. Als Thrombininhibitor, beispielsweise AT III, Hirudin oder Heparin, wird vorzugsweise AT III in einer Konzentration von 0,1 - 1 E/ml Gewebekleber verwendet; in dieser Konzentration verhindert es bei den oben genannten Konzentrationen an Prothrombinfaktoren und Calciumionen eine vorzeitige Fibrinbildung. Andererseits kann diese aber begünstigt werden, wenn dies von der Indikation her wünschenswert erscheint. Zu diesem Zweck kann man den lyophilisierten Kleber in einer höheren Caciumionen-Endkonzentration als 0,5 - 1 mmol/l lösen. Dies erfordert allerdings eine schnelle Anwendung nach der Rekonstitution.

Zur Herstellung des Klebers werden die Wirksubstanzen zunächst in möglichst aktiver und gegebenenfalls pasteurisierter löslicher Form hergestellt. Die Fibrinogenkonzentration sollte möglichst hoch sein, da die Klebefestigkert mit steigender Konzentration zunimmt Das Fibrinogen kann beispielsweise nach der EP 0 103 196 hergestellt und pasteurisiert und nach EP 0 085 923 durch Zusätze vom Typ harnstoff- oder guanidinreste-enthaltende Verbindung auf die gewünschte Löslichkeit gebracht werden. Faktor XIII kann gemäß EP 0 018 561 und die Prothrombinfaktoren können nach EP 0 056 629 pasteurisiert werden.

Die Wirksubstanzen des bevorzugten erfindungsgemäßen Klebers bestehen im Prinzip aus Humanfibrinogen in einer möglichst hohen Konzentration, angereichert mit Faktor XIII und den Faktoren des Prothrombinkomplexes (Faktor II, VII, IX und X), Calciumionen sowie Antithrombin III. Der Gewebeklebstoff enthält ferner einen Inhibitor, der Plasminogenaktivatoren und/oder Plasmin inhibiert und damit das entstandene Fibrin vor einem Abbau durch Plasmin schützt. Dieser Inhibitor kann als Begleitsubstanz des für die Klebung ausgewählten Fibrinogens vorliegen, wird aber vorzugsweise in Form von Aprotinin dem Kleber zugesetzt. Zur Verbesserung der Rekonstitution des Gewebeklebstoffes nach Lyophilisation kann dieser Albumin und/oder Substanzen, die den Harnstoff- oder Guanidinrest enthalten, sowie gegebenenfalls auch eine Aminosäure mit hydrophober Seitenkette oder eine wasserlösliche Fettsäure und weiterhin Heparin enthalten.

Für die Zubereitung des Gewebeklebstoffes kommt vorzugsweise gereinigtes Fibrinogen in Betracht, das Faktor XIII bereits enthält. Daneben eignet sich aber auch Kryopräzipitat, in dem erfahrungsgemäß neben Faktor XIII auch alpha₂-Antiplasmin und Humanalbumin vorliegen. Ein Klebstoff vergleichbarer Wirkung kann aber auch aus den einzelnen Komponenten durch Mischen hergestellt werden, nämlich: Humanfibrinogen, Faktor XIII (aus Plasma oder auch Plazenta), Plasmin-Inhibitoren, Prothrombinfaktoren (Faktor II, VII, IX und X), Alburnin, AT III und Calciumionen, einer Verbindung mit einem Harnstoff- oder Guanidinrest oder einer Aminosäure mit hydrophober Seitenkette oder einer wasserlösliche Fettsäure.

Das Gemisch der Wirkstoffe für den Kleber liegt vorteilhafter Weise in fester Form, vorzugsweise als Lyophilisat vor.

Der beanspruchte Gewebekleber enthält folgende Mengen gegebenenfalls pasteurisierter Wirkstoffe bezogen auf 1 ml Anwendungslösung:
65 - 115, bevorzugt 70 - 90 mg Humanfibrinogen
40 - 80 E Faktor XIII
10 - 30 IE PPSB (Prothrombinfaktoren),
   bezogen auf F II (Prothrombin)
0 - 10 000 KIE Aprotinin
0,01 - 50, bevorzugt 0,1 - 1 IE Antithrombin III und
0,1 - 5, bevorzugt 0,5 - 1 mmol/l Calciumionen, vorzugsweise als Calciumchlorid.

Als Stabilisatoren und Lösungsvermittler kann der Kleber beispielsweise Glutaminat, L-Isoleucin, L-Arginin oder Humanalbumin enthalten.

Das Gemisch der festen Wirkstoffe des Gewebeklebstoffs kann in kurzer Zeit bei 20 °C in Wasser oder einem calciumionenhaltigen Lösungsmittel gelöst werden. Der Kleber entwickelt innerhalb einer genügend kurzen Zeit eine hohe Klebefestigkeit, wenn er auf das zu verbindende oder zu klebende Gewebe gebracht wird, ohne daß als zweite Komponente Thrombin erforderlich ist.

Demzufolge kann der Kleber mit einer einzigen Spritze in flüssiger Form aufgetragen werden, so daß Zubereitung, Handhabung und Anwendung einfach sind. Der Kleber ist verträglich und wird voll resorbiert.

Dieser Einkomponenten-Kleber unterscheidet sich weder in der Klebezeit noch in der Reißfestigkeit von einem Zwei-Komponenten-Kleber, wie in Klebeversuchen am Modell der Rattenhaut-Stanzwunde und bei der Klebung von kleinen Darmanastomosen beim Schwein gefunden wurde. Durch die Wahl einer optimalen Calciumionen-Konzentration der Lösung, mit der man die lyophilisierten Wirkstoffe löst, kann man die Klebefestigkeit noch verstärken.

Die folgenden Beispiele sollen die Erfindung erläutern.

### Beispiel 1

Zur Herstellung von 500 ml Gewebekleber wurden 580 g pasteurisiertes, hochgereinigte Humanfibrinogen, das 2 Calciumionen pro Mol enthielt und nach EP 0 103 196 gewonnen worden war, in 580 ml Puffer, pH 7,5 (Dialysepuffer) mit folgender Zusammensetzung gelöst:
0,05 mol/l NaCl
0,005 mol/l Tri-Natriumcitrat
0,3 g/100 ml L-Argininmonohydrochlorid

Die so erhaltene Fibrinogenlösung wurde zweimal 16 Stunden und einmal 8 Stunden gegen je 36 l Puffer derselben Zusammensetzung bei +4°C dialysiert. Das Volumen der Fibrinogenlösung betrug nach der Dialyse 1,53 l. Die Lösung wurde anschließend 20 min bei 8000 g zentrifugiert. Die optische Dichte der Lösung, gemessen bei 280 nm, betrug 47. Diese Lösung wurde mit dem obigen Puffer, dem die anderen Kleberkomponenten zugesetzt worden waren (s. unten), auf eine optische Dichte (280 nm) von 35 bis 37, d.h. auf etwa 20 g/l Humanfibrinogen verdünnt.

Im einzelnen wurde dabei folgendermaßen verfahren: Die dialysierte und zentrifugierte Humanfibrinogenlösung (1,53 l) wurde bei 35 bis 37°C nacheinander über Filter der Porenweiten 0,65 und 0,2 µ filtriert. Zu der erhaltenen sterilen Fibrinogenlösung wurden 117,5 ml pasteurisiertes F XIII-Konzentrat, enthaltend 350 E F XIII/ml physiologischer Kochsalzlösung, durch ein Filter mit 0,2 µ Porenweite filtiert.

Dann wurden 515 000 KIE (Kallikrein Inhibitor Einheiten) Aprotinin, 5,13 g Na-Glutaminat, 33,9 ml einer 20 g/100 ml enthaltenden Humanalbuminlösung, 6,78 g Isoleucin und 10 000 E pasteurisiertes PPSB-Konzentrat (Prothrombinfaktoren-Konzentrat; Aktivität in Einheiten bezogen auf F II), 100 E AT III (pasteurisiert) und 3,1 ml 0,1 mol/l CaCl₂-Lösung zugesetzt.

Anschließend wurde das Volumen mit Dialysepuffer auf 407,5 ml aufgefüllt. Diese Lösung wurde über ein Filter mit 0,2 µ Porenweite zu der F XIII-haltigen Fibrinogenlösung filtriert. Es wurden etwa 2 055 ml Lösung von pH 7,5 und folgender Zusammensetzung erhalten:
Humanfibrinogen 2,0 g/100 ml
Humanalbumin 0,33 g/100 ml
Arginin 0,3 g/100 ml
Na-Glutaminat 2,5 g/l
Isoleucin 3,3 g/l
Faktor XIII 20 000 E/l
Aprotinin 250 000 KIE/l
Prothrombin 5000 E/l (als F II berechn.)
Antithrombin III 50 E/l
NaCl 0,05 mol/l
tri-Natriumcitrat 0,005 mol/l
Calciumchlorid 0,15 mmol/l.

Die sterile Lösung wurde zu 4 ml abgefüllt und lyophilisiert. Es wurde ein farbloses, schnell lösliches Lyophilisat erhalten.

Zur Anwendung als Gewebekleber wurde eine Abfullung in 1 ml Lösungsmittel aufgenommen.

### Beispiel 2

Zur Herstellung von 125 ml Gewebekleber wurden 100 g einer pasteurisierten, hochgereinigten Humanfibrinogenfraktion, gewonnen nach EP 0 103 196, in 112 ml Dialysepuffer gelöst und dreimal gegen je 10 l desselben Puffers wie in Beispiel 1 dialysiert. Das Volumen der konzentrierten Fibrinogenlösung betrug nach Dialyse 269 ml. Sie wurde 20 min bei 8000 g zentrifugiert Die optische Dichte der Lösung bei 280 nm betrug 69,2. Mit einem Puffer, dessen Zusammensetzung dem Dialysepuffer entspricht und dem alle anderen Kleberbestandteile beigemischt sind, wurde die Lösung so verdünnt, daß eine optische Dichte bei 280 nm von 35 - 37, bezogen auf das Fibrinogen, erreicht wurde.

Dabei wurde wie folgt verfahren:
127 ml vorgelegtem Puffer wurden 8,2 ml Humanalbumin-Lösung, die 20 g Humanalbumin pro 100 ml Lösung enthielt, 6 ml Aprotinin mit 125.000 KIE und 50 ml Prothrombinkonzentrat, enthaltend 2.500 E Faktor II und 25 E AT III zugefügt. Darin wurden 1,65 g L-Isoleucin und 1,25 g Na-Glutaminat gelöst und anschließend 40 ml F XIII-Konzentrat, enthaltend 10.000 E F XIII zugefügt, homogen gemischt und der pH-Wert der Lösung auf pH 7,5 korrigiert.

Anschließend wurde diese Lösung mit der oben beschriebenen Fibrinogenlösung und 0,75 ml einer 0,1 molaren CaCl₂-Lösung homogen gemischt und bei 35 - 37° C über ein Filter der Porenweite von 0,2 µ sterilfiltriert. Es wurden etwa 500 ml sterile Lösung der Zusammensetzung wie im Beispiel 1 erhalten. Diese Lösung wurde in 4-ml-Mengen abgefüllt und lyophilisiert. Es wurde ein farbloses, schnell lösliches Lyophilisat erhalten.

Zur Anwendung als Gewebekleber wird eine Abfüllung in 1 ml Lösungsmittel aufgenommen.

## Patentansprüche (Patentansprüche für folgende(n) Vertragsstaat(en): AT, BE, CH, DE, FR, GB, IT, LI, LU, NL, SE)

1. Gewebekleber, enthaltend ein Fibrinogen, F XIII, einen Thrombininhibitor, Prothrombinfaktoren und Calciumionen sowie gegebenenfalls einen Plasmininhibitor, dadurch gekennzeichnet, daß er in 1 ml Anwendungslösung
65 - 115 mg Humanfibrinogen,
40 - 80 E Faktor XIII,
10 - 30 IE PPSB (Prothrombinfaktoren), bezogen auf F II (Prothrombin),
0,01 - 50 IE Antithrombin III und
0,1 - 5 mmol/l Calciumionen enthält.

2. Gewebekleber nach Anspruch 1, dadurch gekennzeichnet, daß er lyophilisiert ist.

3. Gewebekleber nach Anspruch 1, dadurch gekennzeichnet, daß die in ihm enthaltenen Wirksubstanzen pasteurisiert sind.

4. Gewebekleber nach Anspruch 1, dadurch gekennzeichnet, daß er in Lösung
70 - 90 mg/ml Humanfibrinogen,
10 - 30 IE/ml (bezogen auf F II) Prothrombinfaktoren,
0,5 - 1 mmol/l Calciumionen und
0,1 - 1 IE/m l Antithrombin III enthält.

5. Gewebekleber nach Anspruch 1, dadurch gekennzeichnet, daß er in 1 ml Lösung 1 - 10 000 KIE Aprotinin enthält.

6. Verfahren zur Herstellung eines Gewebeklebers nach Anspruch 1, dadurch gekennzeichnet, daß man zu einer gegebenenfalls pasteurisierten und gegebenenfalls sterilfiltrierten wässrigen isotonischen Lösung von pH 7,5, die mindestens 16 g/l Humanfibrinogen, 2 g Atome Calcium/mol Fibrinogen und 1 - 6 g/l L-Argininmonohydrochlorid enthält, so viel einer gegebenenfalls pasteurisierten Lösung von Human-Faktor XIII, von Human-Albumin, von Prothrombinkonzentrat, von Antithrombin III und von Aprotinin sowie gegebenenfalls Na-Glutaminat und Isoleucin zugibt, daß die lyophilsierte Lösung nach Rekonstitution in ¼ des Abfüllvolumens diese Stoffe in folgenden Konzentrationsbereichen enthält:
65 - 115 mg/ml Humanfibrinogen,
40 - 80 E/ml Faktor XIII,
4 - 40 mg/ml Human-Albumin,
10 - 30 IE/ml PPSB (Prothrombinfaktoren), bezogen auf F II (Prothrombin),
0,01 - 50 IE/ml Antithrombin III
und gegebenenfalls 1 - 10 000 KIE Aprotinin/ml
und 0 - 20 g/l Na-Glutaminat
und 0 - 20 g/l Isoleucin.

## Patentansprüche (Patentansprüche für folgende(n) Vertragsstaat(en): ES, GR)

1. Verfahren zur Herstellung eines Gewebeklebers, dadurch gekennzeichnet, daß man zu einer gegebenenfalls pasteurisierten und gegebenenfalls sterilfiltrierten wässrigen isotonischen Lösung von pH 7,5, die mindestens 16 g/l Humanfibrinogen, 2 g Atome Calcium/mol Fibrinogen und 1 - 6 g/l L-Argininmonohydrochlorid enthält, so viel einer gegebenenfalls pasteurisierten Lösung von Human-Faktor XIII, von Human-Albumin, von Prothrombinkonzentrat, von Antithrombin III und von Aprotinin sowie gegebenenfalls Na-Glutaminat und Isoleucin zugibt, daß die lyophilisierte Lösung nach Rekonstitution in ¼ des Abfüllvolumens diese Stoffe in folgenden Konzentrationsbereichen enthält:
65 - 115 mg/ml Humanfibrinogen,
40 - 80 E/ml Faktor XIII,
4 - 40 mg/ml Human-Albumin,
10 - 30 IE/ml PPSB (Prothrombinfaktoren), bezogen auf FII (Prothrombin),
0,01 - 50 IE/ml Antithrombin III
und gegebenenfalls 1 - 10 000 KIE Aprotinin/ml
und 0 - 20 g/l Na-Glutaminat
und 0 - 20 g/l Isoleucin.

## Claims (Claims for the following Contracting State(s): AT, BE, CH, DE, FR, GB, IT, LI, LU, NL, SE)

1. A tissue adhesive containing a fibrinogen, F XIII, a thrombin inhibitor, prothrombin factors and calcium ions and, where appropriate, a plasmin inhibitor, which contains, in 1 ml of solution for use,
65 - 115 mg of human fibrinogen,
40 - 80 U of factor XIII,
10 - 30 IU of PPSB (prothrombin factors), based on F II (prothrombin),
0.01 - 50 IU of antithrombin III and
0.1 - 5 mmol/l calcium ions.

2. A tissue adhesive as claimed in claim 1, which is freeze-dried.

3. A tissue adhesive as claimed in claim 1, wherein the active substances it contains are pasteurized.

4. A tissue adhesive as claimed in claim 1, which contains in solution
70 - 90 mg/ml human fibrinogen,
10 - 30 IU/ml (based on F II) prothrombin factors,
0.5 - 1 mmol/l calcium ions and
0.1 - 1 IU/ml antithrombin III.

5. A tissue adhesive as claimed in claim 1, which contains, in 1 ml of solution, 1 - 10,000 KIU of aprotinin.

6. A process for the production of a tissue adhesive as claimed in claim 1, which comprises addition to an aqueous isotonic solution which has a pH of 7.5, which has been, where appropriate, pasteurized and, where appropriate, sterilized by filtration, and which contains at least 16 g/l human fibrinogen, 2 g atoms of calcium/mol of fibrinogen and 1 - 6 g/l L-arginine monohydrochloride, of sufficient of a solution, which has, where appropriate, been pasteurized, of human factor XIII, of human albumin, of prothrombin concentrate, of antithrombin III and of aprotinin and, where appropriate, Na glutamate and isoleucine that the freeze-dried solution contains, after reconstitution in ¼ of the volume dispensed into the container, these substances in the following concentration ranges:
65 - 115 mg/ml human fibrinogen,
40 - 80 U/ml factor XIII,
4 - 40 mg/ml human albumin,
10 - 30 IU/ml PPSB (prothrombin factors), based on F II (prothrombin),
0.01 - 50 IU/ml antithrombin III
and, where appropriate, 1 - 10,000 KIU of aprotinin/ml
and 0 - 20 g/l Na glutamate
and 0 - 20 g/l isoleucine.

## Claims (Claims for the following Contracting State(s): ES, GR)

1. A process for the production of a tissue adhesive, which comprises addition to an aqueous isotonic solution which has a pH of 7.5, which has been, where appropriate, pasteurized and, where appropriate, sterilized by filtration, and which contains at least 16 g/l human fibrinogen, 2 g atoms of calcium/mol of fibrinogen and 1 - 6 g/l L-arginine monohydrochloride, of sufficient of a solution, which has, where appropriate, been pasteurized, of human factor XIII, of human albumin, of prothrombin concentrate, of antithrombin III and of aprotinin and, where appropriate, Na glutamate and isoleucine that the freeze-dried solution contains, after reconstitution in ¼ of the volume dispensed into the container, these substances in the following concentration ranges:
65 - 115 mg/ml human fibrinogen,
40 - 80 U/ml factor XIII,
4 - 40 mg/ml human albumin,
10 - 30 IU/ml PPSB (prothrombin factors), based on F II (prothrombin),
0.01 - 50 IU/ml antithrombin III
and, where appropriate, 1 - 10,000 KIU of aprotinin/ml
and 0 - 20 g/l Na glutamate
and 0 - 20 g/l isoleucine.

## Revendications (Revendications pour l'(les) Etat(s) contractant(s) suivant(s): AT, BE, CH, DE, FR, GB, IT, LI, LU, NL, SE)

1. Colle pour tissu renfermant du fibrinogène, le facteur F XIII, un inhibiteur de la thrombine, des facteurs de la prothrombine et des ions de calcium ainsi qu'éventuellement un inhibiteur de plasmine, caractérisée en ce qu'elle contient, dans 1 ml de solution,
65-115 mg de fibrinogène humain,
40-80 U de facteur XIII,
10-30 UI de PPSB (facteurs de la prothrombine) calculé par rapport à F II (prothrombine),
0,01-50 UI d'antithrombine III, et
0,1-5 mmole/l d'ions calcium.

2. Colle pour tissu selon la revendication 1, caractérisée en ce qu'elle est lyophilisée.

3. Colle pour tissu selon la revendication 1, caracterisée en ce que toutes les substances actives qu'elle contient sont pasteurisées.

4. Colle pour tissu selon la revendication 1, caractérisée en ce qu'elle contient en solution :
70-90 mg/ml de fibrinogène humain,
10-30 UI/ml (calcule par rapport à F II de facteurs de la prothrombine),
0,5-1 mmole/l d'ions calcium, et
0,1-1 UI/ml d'antithrombine III.

5. Colle pour tissu selon la revendication 1, caractérisée en ce qu'elle contient 1-10 000 KUI d'aprotinine.

6. Procédé pour la préparation d'une colle pour tissu selon la revendication 1, caractérisé en ce que l'on ajoute à une solution isotonique aqueuse éventuellement pasteurisée et éventuellement filtrée à l'état stérile, de pH 7,5 et contenant au moins 16 g/l de fibrinogène humain, 2g d'atomes de calcium par mole de fibrinogène et 1-6 g/l de monochlorhydrate de L-arginine, une quantité suffisante d'une solution éventuellement pasteurisée de facteur XIII humain, d'albumine humaine, de concentré de prothrombine, d'antithrombine III et d'aprotinine ainsi qu'éventuellement de glutaminate de sodium et d'isoleucine pour que la solution lyophilisée contienne ces substances aux concentrations suivantes, après reconstitution dans ¼ du volume de conditionnement :
65-115 mg/ml de fibrinogène humain,
40-80 U/ml de facteur XIII,
4-40 mg/ml d'albumine humaine,
10-30 UI/ml de PPSB (facteurs de la prothrombine), calculé par rapport à F II (prothrombine),
0,01-50 UI/ml d'antithrombine III,
et éventuellement 1-10 000 KUI d'aprotinine/ml,
et 0-20 g/ml de glutaminate de sodium, et
0-20 g/l d'isoleucine.

## Revendications (Revendications pour l'(les) Etat(s) contractant(s) suivant(s): ES, GR)

1. Procédé pour la préparation d'une colle pour tissu, caractérisé en ce que l'on ajoute à une solution isotonique aqueuse éventuellement pasteurisée et éventuellement filtrée à l'état stérile, de pH 7,5 et contenant au moins 16 g/l de fibrinogène humain, 2 g d'atomes de calcium par mole de fibrinogène et 1-6 g/l de monochlorhydrate de L-arginine, une quantité suffisante d'une solution éventuellement pasteurisée de facteur XIII humain, d'albumine humaine, de concentré de prothrombine, d'antithrombine III et d'aprotinine pour que la solution lyophilisée renferme ces substances aux concentrations suivantes, après reconstitution dans ¼ du volume de conditionnement :
65-115 mg/ml de fibrinogène humain,
40-80 U/ml de facteur XIII,
4-40 mg/ml d'albumine humaine,
10-30 UI/ml de PPSB (facteurs de la prothrombine) calculé par rapport à F II (prothrombine),
0,01-50 UI/ml d'antithrombine III,
et éventuellement 1-10 000 KUI d'aprotinine/ml,
et 0-20 g/l de glutaminate de sodium,
et 0-20 g/l d'isoleucine.
